Europäisches Patentamt

(19) European Patent Office

Office européen des brevets

(11) Veröffentlichungsnummer: **0 223 120
B1**

(12) EUROPÄISCHE PATENTSCHRIFT

(45) Veröffentlichungstag der Patentschrift:
31.01.90

(51) Int. Cl.⁴: **A61F 7/00**

(21) Anmeldenummer: 86114950.8

(22) Anmeldetag: 28.10.86

(54) Vorrichtung zur Durchführung der Kryotherapie am ganzen Körper.

(30) Priorität: 22.11.85 DE 3541332
23.07.86 DE 3624822

(43) Veröffentlichungstag der Anmeldung:
27.05.87 Patentblatt 87/22

(45) Bekanntmachung des Hinweises auf die Patenterteilung:
31.01.90 Patentblatt 90/5

(84) Benannte Vertragsstaaten:
AT BE CH FR GB IT LI NL SE

(56) Entgegenhaltungen:
DE-A- 2 340 150
JP-U-56 168 125
US-A- 3 587 577
US-A- 3 908 655

(73) Patentinhaber: MESSER GRIESHEIM GMBH, Hanauer
Landstrasse 330, D-6000 Frankfurt/Main 1(DE)

(72) Erfinder: Donnerhack, Andreas, Dr., Bismarckstrasse 4,
D-4150 Krefeld(DE)
Erfinder: Thoma, Klemens, Am Kleckers 22,
D-4150 Krefeld-Hüls 29(DE)
Erfinder: Volker, Wolfgang, Pastorsbusch 35,
D-4154 Tönisvorst 1(DE)
Erfinder: Gallmeister, Rolf-Dieter, Uhlandstrasse 14,
D-7880 Bad Säckingen(DE)
Erfinder: Stratz, Thomas, Dr., Purkersdorferstrasse 49,
D-7880 Bad Säckingen(DE)
Erfinder: Lammers, Ludwig, Dr., Saalburgweg 3,
D-6270 Idstein(DE)

## Beschreibung

Die Erfindung betrifft eine Vorrichtung zur Durchführung der Kryotherapie am ganzen Körper mit einem kalten Behandlungsgas, nach dem Oberbegriff des Anspruches 1.

Neben der seit einigen Jahren durchgeführten lokalen Kryotherapie mit einem kalten Behandlungsgas zur Behandlung rheumatischer Erkrankungen wird bei bestimmten Krankheitsformen auch eine Kryotherapie am ganzen Körper durchgeführt. Hierbei wird mit Hilfe von flüssigem Stickstoff Luft in Wärmeaustauschern abgekühlt und in einen geschlossenen Behandlungsraum eingeleitet. Dieser als Kammer oder Kabine ausgebildete Behandlungsraum besitzt Wände aus isolierendem Material und Anschlüsse zur Zufuhr und Ableitung des Behandlungsgases. Einen derartigen Behandlungsraum zeigt beispielsweise das japanische Gebrauchsmuster Nr. 168 125/81. Dieses Konzept findet jedoch sowohl bei Ärzten als auch bei Patienten wenig Anklang. Die Gründe dafür sind vielfältig. Die Patienten beanstanden den fehlenden direkten Kontakt zum Arzt, da während der Behandlung nur ein indirekter Kontakt durch Fenster und über Sprecheinrichtungen möglich ist. Die starke Nebelbildung in der Kammer verstärkt noch diesen Eindruck des mangelnden direkten Kontaktes. Nachteilig ist auch die Abkühlung im Kopfbereich des Patienten. Außerdem muß durch besondere Maßnahmen das Einatmen kalter Luft verhindert werden. Derartige Kammern erfordern auch hohe Investitionskosten. Wegen der langen Anlaufzeiten besteht die Notwendigkeit zum Dauerbetrieb, wodurch verhältnismäßig hohe Betriebskosten entstehen. Die Patientenüberwachung während der Behandlung ist aufwendig.

Der Erfindung liegt daher die Aufgabe zugrunde, eine Vorrichtung zur Durchführung der Kryotherapie am ganzen Körper mit einem kalten Behandlungsgas zu schaffen, welche einen direkten Kontakt zwischen Arzt und Patienten während der Behandlung ermöglicht, den Kopf des Patienten frei läßt, es dem Patienten jederzeit ermöglicht, die Vorrichtung zu verlassen und welche wegen kurzer Anlaufzeiten keinen Dauerbetrieb erforderlich macht.

Ausgehend von dem im Oberbegriff des Anspruches 1 berücksichtigten Stand der Technik ist diese Aufgabe erfindungsgemäß mit den im kennzeichnenden Teil des Anspruches 1 angegebenen Merkmalen gelöst.

Vorteilhafte Weiterbildungen der Erfindung sind in den Unteransprüchen angegeben.

Durch die Zuordnung von Bedienpult und Halbschale wird einerseits ein Behandlungsraum geschaffen, in welchem der Körper des Patienten wirkungsvoll mit kaltem Behandlungsgas beaufschlagt werden kann. Andererseits ist nicht nur ein Blick- und Sprechkontakt zwischen Arzt und Patienten während der Behandlung möglich, sondern der Arzt kann auch direkt eingreifen, um beispielsweise den Patienten zu positionieren. Trotzdem kann der Patient, wenn er dies aus irgendwelchen Gründen wünscht, jederzeit sofort die Vorrichtung verlassen. Auf die Zufuhr von Behandlungsgas durch das Bedienpult kann insbesondere dann verzichtet werden, wenn der Druck des aus den Öffnungen in den seitlichen Teilen der Halbschale austretenden Behandlungsgases genügend groß ist, um den durch die Halbschale gebildeten Behandlungsraum wirksam zu beaufschlagen. Hierzu ist es zweckmäßig, die Öffnungen zur Zufuhr des Behandlungsgases in vertikalen Düsenleisten anzuordnen, die in der Öffnungsfläche der Halbschale aufeinanderzu verfahrbar und um ihre vertikale Achse drehbar sind. Dies ermöglicht auch ein individuelles Eingehen auf die jeweilige Körpergröße und Körperform des Patienten. Generell werden zumindest die hochgelegenen Austrittsöffnungen für das Behandlungsgas verschließbar gestaltet, um zu ermöglichen, auch bei kleinen Patienten den Kopfbereich nicht mit Behandlungsgas zu beaufschlagen.

Ein Ausführungsbeispiel der Erfindung soll anhand der beigefügten Zeichnungen erläutert werden.

Es zeigen:

Fig. 1 einen Längsschnitt durch eine Vorrichtung zur Durchführung der Kryotherapie,

Fig.2 die Ansicht A-A in Fig.1,

Fig.3 einen Querschnitt durch die Vorrichtung entlang der Linie B-B in Fig.1,

Fig.4 den Schnitt durch ein Hohlprofil mit schwenkbaren Öffnungen,

Fig.5 eine Einrichtung zur Erzeugung des Behandlungsgases, welche einen kostengünstigen Leerlaufbetrieb der Vorrichtung ermöglicht.

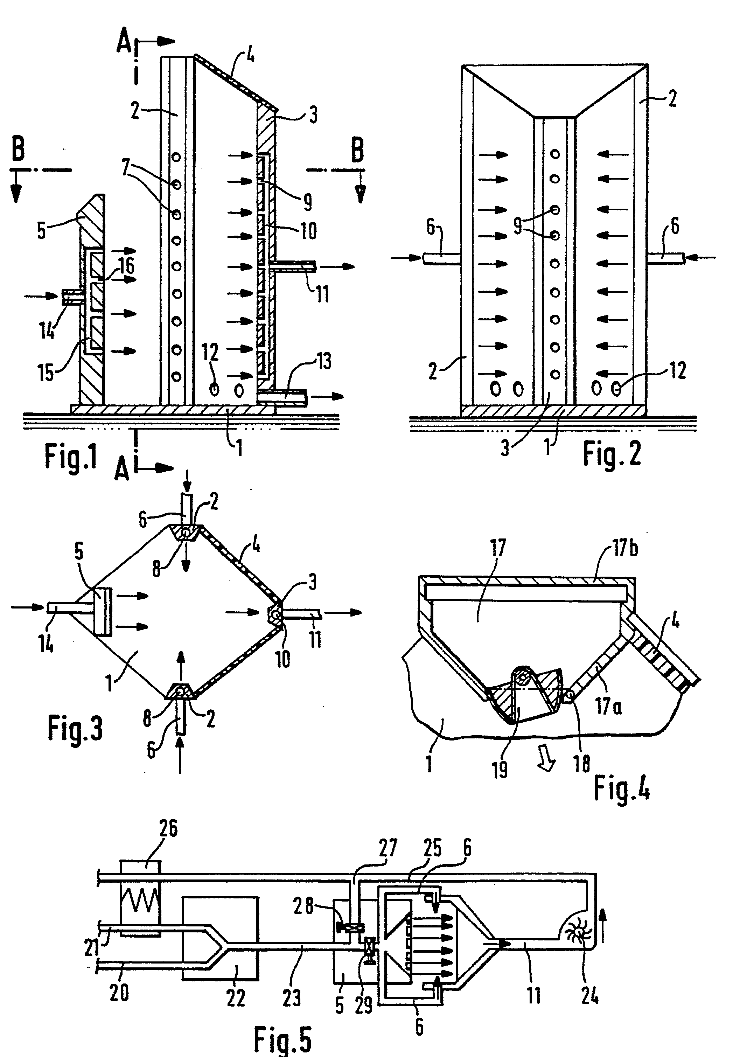

Die in den Figuren 1 bis 3 dargestellte Vorrichtung ist auf einer Grundplatte 1 montiert. Den eigentlichen Behandlungsraum bildet eine Halbschale, die im wesentlichen gebildet wird aus zwei seitlichen Profilen 2 und einem hinteren Profil 3. Zwischen den Profilen 2, 3 sind Wände 4 aus isolierendem Material befestigt. In definiertem Abstand ist vor der Öffnung der Halbschale ein Bedienpult 5 aufgestellt. Der Abstand wird so festgelegt, daß sich vor und in der Halbschale eine optimale Strömung des Behandlungsgases einstellt. Die Strömungsrichtung des Behandlungsgases ist durch Pfeile angegeben.

Die Zufuhr des kalten Behandlungsgases erfolgt durch Anschlüsse 6 in die seitlichen Profile 2. Hier wird es mittels einer Rohrleitung 8 auf die einzelnen Öffnungen 7 verteilt, durch welche es in die Halbschale eindringt. Durch entsprechende Öffnungen 9 im hinteren Profil 3, eine Rohrleitung 10 und einen Anschluß 11 wird das Behandlungsgas aus der Halbschale abgezogen.

Die Profile 2, 3 sind zweckmäßigerweise Hohlprofile, die nach Verlegung der Rohrleitungen 8, 10 ausgeschäumt sein können. Außerdem sind in der Nähe der Grundplatte 1 in den Wänden 4 weitere Öffnungen 12 zur Ableitung des Behandlungsgases vorgesehen. Diese Öffnungen 12 sind durch eine nicht dargestellte Rohrleitung miteinander verbunden, welche in den Anschluß 13 mündet.

Eine weitere Zufuhr von kaltem Behandlungsgas erfolgt durch das Bedienpult 5, in welchem ein entsprechender Anschluß 14 vorgesehen ist. An den Anschluß 14 schließt sich eine Rohrleitung 15 an, durch welche das kalte Behandlungsgas schließlich zu den Öffnungen 16 gelangt, aus denen es in Richtung der Halbschale austritt. Der Patient betritt und verläßt die Vorrichtung durch den zwischen Bedienpult 5 und Halbschale gebildeten Durchlaß. Auch während der Behandlung kann er die Vorrichtung sofort verlassen, wenn er dies wünscht. Der Arzt kann auch bei laufender Behandlung jederzeit den Patienten berühren und ihn beispielsweise anders positionieren.

Es ist vorteilhaft, wenn die Öffnungen 7, 9, 12 und 16 einzeln verschließbar sind, um bestimmte erwünschte Strömungsformen in der Halbschale zu erreichen. Hierzu ist es auch vorteilhaft, wenn insbesondere die Öffnungen 7 in den seitlichen Profilen 2 schwenkbar angeordnet sind, so daß dem einströmenden kalten Behandlungsgas verschiedene Strömungsrichtungen aufgeprägt werden können. Es ist auch vorteilhaft, die Öffnungen 7 und 9 in Düsenleisten anzuordnen, die in verschiedener Höhe in den Profilen 2 und 3 angeordnet werden können. Eine solche Ausführungsform zeigt Fig. 4.

Das in Fig. 4 im Schnitt dargestellte Hohlprofil 17 entspricht dem seitlichen Profil 2 in den Figuren 1 bis 3. Das Hohlprofil 17 ist aus dem Oberteil 17a und dem Unterteil 17b zusammengesetzt. In das Oberteil 17a ist eine Düsenleiste 18 eingesetzt. Die Düsenleiste 18 kann je nach Bedarf in verschiedener Höhe des Hohlprofils 17 eingesetzt werden. In der Düsenleiste 18 befindet sich eine Anzahl nach allen Seiten schwenkbarer Düsen 19, welche den Öffnungen 7 in Fig. 1 entsprechen. Die Düsen 19 können auch einzeln geschlossen werden. Derartige Düsen 19 sind an sich aus der Belüftungstechnik bekannt.

Das Bedienpult 5 erlaubt dem Therapeuten die Einstellung und Überwachung der wichtigsten Behandlungsparameter.

Die Möglichkeit, einzelne Öffnungen 7 bzw. Düsen 19 zu verschließen, ist wichtig, um die Vorrichtung Patienten mit unterschiedlicher Körpergröße anzupassen, oder um gezielt nur bestimmte Teile des Körpers zu behandeln. Durch die gezielte Absaugung des Behandlungsgases, vor allem in Bodennähe, wird eine starke Nebelbildung vermieden. Die Möglichkeit, daß der Patient die Vorrichtung jederzeit einfach verlassen kann, ohne durch eine Tür oder Schleuse behindert zu sein, vermittelt dem Patienten das Gefühl, der Behandlung nicht ausgeliefert zu sein. Die Akzeptanz der Therapie durch den Patienten wird damit verbessert.

Für die Bildung des kalten Behandlungsgases können alle hierfür gebräuchlichen Einrichtungen und Gaszusammensetzungen verwendet werden. Vorteilhaft ist die Bildung des Behandlungsgases durch Vermischen von trockener Luft mit einem kalten verflüssigten Gas, vorzugsweise Stickstoff. Ebenso kann das kalte Behandlungsgas auch durch Abkühlen von getrockneter Luft in einem Wärmetauscher mit Hilfe eines kalten verflüssigten Gases, vorzugsweise Stickstoff, erzeugt werden.

Eine hierzu geeignete Einrichtung ist in Fig.5 dargestellt. Durch die Leitung 20 wird flüssiger Stickstoff und durch die Leitung 21 trockene Luft in das Mischgerät 22 eingespeist. Das im Mischgerät 22 gebildete kalte Behandlungsgas strömt durch die Leitung 23 in das Bedienpult 5 und die Anschlüsse 6 in den erfindungsgemäß als offene Halbschale ausgebildeten Behandlungsraum. Die Richtung der Gasströmung ist wiederum durch nicht bezeichnete Pfeile dargestellt. Durch den Anschluß 11 wird das Behandlungsgas mittels des Absauggebläses 24 über die Leitung 25 durch den Wärmetauscher 26 geleitet. Im Wärmetauscher 26 gibt es seine Kälte an die eintretende Luft in Leitung 21 ab. Auf diese Weise wird bei Betrieb der erfindungsgemäßen Vorrichtung die Kälte optimal ausgenutzt. Die Absaugung bewirkt eine gerichtete Strömung und dient der Kälterückgewinnung.

Wenn kein Patient behandelt wird, die Vorrichtung aber betriebsbereit gehalten werden soll, wird die Anlage auf Leerlaufbetrieb umgeschaltet. Bei Leerlauf strömt das kalte Behandlungsgas unmittelbar durch die Leitung 27 in den Wärmetauscher 26 zurück. Die Umschaltung auf Leerlaufbetrieb erfolgt durch Betätigen der Ventile 28 und 29. Der Leerlaufbetrieb wird zweckmäßigerweise mit einer geringeren Gasmenge aufrechterhalten. Er soll lediglich das Kalthalten der Kaltwinderzeugungsanlage und der Zuleitungen gewährleisten. Die erfindungsgemäße Vorrichtung ist somit auch in Behandlungspausen betriebsbereit und bleibt kurzfristig einsetzbar. Außer der Energieeinsparung hat eine solche Betriebsweise auch den Vorteil, daß der Patient nicht unter Kühlbedingungen positioniert werden muß.

Die Vorrichtung ist mit den üblichen Sicherheitseinrichtungen ausgerüstet, die jedoch nicht dargestellt sind. Es handelt sich hierbei im wesentlichen um einen Sauerstoffsensor im Kopfbereich des Patienten, der bei Sauerstoffmangel in der Atemluft automatisch eine Abschaltung des Kühlbetriebes auslöst. Des weiteren können Infrarotsonden vorgesehen werden, die eine berührungslose Temperaturüberwachung der Hautoberfläche des Patienten ermöglichen.

## Patentansprüche

1. Vorrichtung zur Durchführung der Kryotherapie am ganzen Körper mit einem kalten Behandlungsgas, bestehend aus einem Behandlungsraum aus isolierendem Material zur Aufnahme des Patienten, wobei der Behandlungsraum Anschlüsse (6,14,11) zur Zufuhr und Ableitung des Behandlungsgases besitzt,
dadurch gekennzeichnet,
daß der Behandlungsraum als offene Halbschale ausgebildet ist, in deren hinterem Teil Öffnungen (9) zur Ableitung des Behandlungsgases und in deren seitlichen Teilen Öffnungen (7) zur Zufuhr des Behandlungsgases in das Innere der Halbschale angeordnet sind.

2. Vorrichtung nach Anspruch 1,
dadurch gekennzeichnet,
daß vor der Öffnung der Halbschale in definiertem Abstand ein Bedienpult (5) befestigt ist, welches in die Halbschale gerichtete Öffnungen (16) zur Zufuhr des Behandlungsgases aufweist.

3. Vorrichtung nach Anspruch 1 oder 2,
dadurch gekennzeichnet,
daß die Halbschale aus Hohlprofilen (17) mit dazwischen befestigten Wänden (4) gebildet wird und die Öffnungen zur Zufuhr und Ableitung des Behandlungsgases in den Hohlprofilen angeordnet sind.

4. Vorrichtung nach Anspruch 3, dadurch gekennzeichnet, daß die Öffnungen zur Zufuhr und Ableitung des Behandlungsgases in Düsenleisten (18) angeordnet sind, welche in die Hohlprofile eingesetzt sind.

5. Vorrichtung nach Anspruch 4, dadurch gekennzeichnet, daß die Düsenleisten in verschiedener Höhe in die Hohlprofile einsetzbar sind.

6. Vorrichtung nach Anspruch 4 oder 5, dadurch gekennzeichnet, daß die Öffnungen zur Zufuhr der Behandlungsgase als schwenkbare Düsen (19) in den Düsenleisten ausgebildet sind.

7. Vorrichtung nach Anspruch 1 oder 2, dadurch gekennzeichnet, daß die in den seitlichen Teilen der Halbschale befindlichen Öffnungen (7) zur Zufuhr des Behandlungsgases als zwei vertikale, am Rand der Halbschale installierte Düsenleisten ausgebildet sind.

8. Vorrichtung nach Anspruch 7, dadurch gekennzeichnet, daß die Düsenleisten in der Öffnungsfläche der Halbschale aufeinanderzu verfahrbar sind.

9. Vorrichtung nach Anspruch 7 oder 8, dadurch gekennzeichnet, daß die Düsenleisten um ihre vertikale Achse drehbar sind.

10. Vorrichtung nach einem der Ansprüche 1 bis 9, gekennzeichnet durch in Bodennähe befindliche zusätzliche Öffnungen (12) zur Ableitung des Behandlungsgases.

11. Verfahren zum Betreiben der Vorrichtung nach einem der Ansprüche 1 bis 10, bei dem das Behandlungsgas durch Vermischen von trockener Luft mit einem kalten verflüssigten Gas gebildet wird und das aus der Vorrichtung zurückgeführte Behandlungsgas über einen Wärmetauscher (26) geleitet wird, der von warmer trockener Luft zur Bildung des Behandlungsgases durchströmt wird, dadurch gekennzeichnet, daß im Leerlaufbetrieb das Behandlungsgas bereits vor dem Eintritt in das Innere der Vorrichtung umgelenkt und über den Wärmetauscher zurückgeführt wird.

## Claims

1. Apparatus for carrying out crymotherapy over the whole body with a cold treatment gas, consisting of a treatment chamber made of insulating material for accommodating the patient, the treatment chamber having connections (6, 14, 11) for feeding in and removing the treatment gas, characterized in that the treatment chamber is designed as an open half shell, in the rear part of which openings (9) are arranged for removing the treatment gas, and in the lateral parts of which openings (7) are arranged for feeding the treatment gas into the interior of the half shell.

2. Apparatus according to Claim 1, characterized in that there is fixed in front of the opening of the half shell at a defined distance a control console (5) having openings (16) directed into the half shell for feeding in the treatment gas.

3. Apparatus according to Claim 1 or 2, characterized in that the half shell is formed from hollow sections (17) with walls (4) fixed in between them and the openings for feeding in and removing the treatment gas are arranged in the hollow sections.

4. Apparatus according to Claim 3, characterized in that the openings for feeding in and removing the treatment gas are arranged in nozzle strips (18) which are inserted into the hollow sections.

5. Apparatus according to Claim 4, characterized in that the nozzle strips can be inserted into the hollow sections at various levels.

6. Apparatus according to Claim 4 or 5, characterized in that the openings for feeding in the treatment gases are designed as swivellable nozzles (19) in the nozzle strips.

7. Apparatus according to Claim 1 or 2, characterized in that the openings (7) located in the lateral parts of the half shell for feeding in the treatment gas are designed as two vertical nozzle strips installed at the edge of the half shell.

8. Apparatus according to Claim 7, characterized in that the nozzle strips can be moved towards one another in the opening face of the half shell.

9. Apparatus according to Claim 7 or 8, characterized in that the nozzle strips are rotatable about their vertical axis.

10. Apparatus according to one of Claims 1 to 9, characterized by additional openings (12) located in the vicinity of the floor for removing the treatment gas.

11. Method for operating the apparatus according to one of Claims 1 to 10, in which the treatment gas is formed by mixing dry air with a cold liquified gas and the treatment gas returned from the apparatus is fed over a heat exchanger (26), through which warm dry air flows for forming the treatment gas, characterized in that in the idle mode the treatment gas is diverted already before entry into the interior of the apparatus and is returned via the heat exchanger.

## Revendications

1. Dispositif pour l'application de la cryothérapie à l'ensemble du corps, avec un gaz de traitement froid, dispositif constitué d'un espace de traitement en matériau isolant pour recevoir le patient, cet espace de traitement comportant des raccordements (6, 14, 11) pour l'alimentation et l'évacuation du gaz de traitement, dispositif caractérisé en ce que l'espace de traitement revêt la forme d'une demi-coquille ouverte dans la partie arrière de laquelle il est prévu des orifices (9) pour l'évacuation du gaz de traitement et dans les parties latérales de laquelle il est prévu des orifices (7) pour amener le gaz de traitement à l'intérieur de la demi-coquille.

2. Dispositif selon la revendication 1, caractérisé en ce que, en avant de l'ouverture de la demi-coquille et à une distance définie de celle-ci, est fixé un pupitre de service (5), qui comporte des orifices (16) dirigés dans la demi-coquille pour amener du gaz de traitement.

3. Dispositif selon la revendication 1 ou 2, caractérisé en ce que la demi-coquille est constituée par des profilés creux (17) avec des parois (4) fixées entre eux, et les orifices pour l'amenée et l'évacuation du gaz de traitement sont disposés dans ces profilés creux.

4. Dispositif selon la revendication 3, caractérisé en ce que les orifices pour l'amenée et l'évacuation du gaz de traitement sont disposés dans des groupes de buses (18), lesquelles sont mises en place dans les profilés creux.

5. Dispositif selon la revendication 4, caractérisé en ce que les groupes de buses sont susceptibles d'être mis en place à différentes hauteurs dans les profilés creux.

6. Dispositif selon la revendication 4 ou 5, caractérisé en ce que les orifices pour l'amenée des gaz de traitement sont réalisés sous la forme de buses pivotantes (19) dans les groupes de buses.

7. Dispositif selon la revendication 1 ou 2, caractérisé en ce que les orifices (7) se trouvant dans les parties latérales de la demi-coquille pour amener le gaz de traitement, sont réalisés sous la forme de deux groupes de buses verticaux installés sur le bord de la demi-coquille.

8. Dispositif selon la revendication 7, caractérisé en ce que les groupes de buses dans la surface d'ouverture de la demi-coquille peuvent être déplacés les uns par rapport aux autres.

9. Dispositif selon la revendication 7 ou 8, caractérisé en ce que les groupes de buses sont susceptibles de tourner autour de leur axe vertical.

10. Dispositif selon une des revendications 1 à 9, caractérisé en ce qu'il est prévu des orifices supplémentaires (12) au voisinage du fond pour l'évacuation du gaz de traitement.

11. Procédé pour l'exploitation du dispositif selon une des revendications 1 à 10, procédé dans lequel le gaz de traitement est obtenu par mélange d'air sec avec un gaz froid liquéfié, tandis que le gaz de traitement recyclé à partir du dispositif est canalisé à travers un échangeur thermique (26) qui est parcouru par l'air sec chaud pour obtenir le gaz de traitement, procédé caractérisé en ce que, dans le fonctionnement à vide, le gaz de traitement est dévié avant son entrée à l'intérieur du dispositif et recyclé par l'intermédiaire de l'échangeur thermique.

EP 0 223 120 B1

Fig.1

Fig.2

Fig.3

Fig.4

Fig.5